# EUROPÄISCHE PATENTSCHRIFT

(11) **EP 1 344 518 B1**
(45) Veröffentlichungstag und Bekanntmachung des Hinweises auf die Patenterteilung: **02.11.2011**
(21) Anmeldenummer: 03004624.7
(22) Anmeldetag: 03.03.2003
(51) Int. Cl.: A61K 8/44, A61K 8/66, A61Q 19/10, A61Q 5/02

(54) **Kosmetische und pharmazeutische Zubereitungen enthaltend einen oxalkylierten Polyglycerinester**
Cosmetic and pharmaceutical compositions containing oxyalkylated polyglycerol esters
Compositions cosmétiques et pharmaceutiques contenant des esters de polyglycerol oxyalkylés

(30) Priorität: 16.03.2002 DE 10211801
(43) Veröffentlichungstag der Anmeldung: 17.09.2003
(73) Patentinhaber: Clariant Produkte (Deutschland) GmbH, 65929 Frankfurt am Main (DE)
(72) Erfinder: Klug, Peter, Dr., 63762 Grossostheim (DE); Scherl, Franz Xaver, Dr., 84508 Burgkirchen (DE)

(56) Entgegenhaltungen:
- EP-A- 0 264 826
- EP-A- 1 055 407
- WO-A-01/08481
- DE-A1- 2 024 051
- DE-A1- 19 505 178
- DE-C1- 3 239 564
- US-A- 2 679 520
- PATENT ABSTRACTS OF JAPAN Bd. 008, Nr. 034 (C-210), 15. Februar 1984 (1984-02-15) & JP 58 196258 A (NIHON SAAFUAKUTANTO KOGYO KK), 15. November 1983 (1983-11-15)
- DATABASE WPI Section Ch, Week 198631 Derwent Publications Ltd., London, GB; Class A96, AN 1986-199659 XP002369824 & JP 61 130208 A (SUNSTAR KK) 18. Juni 1986 (1986-06-18)
- DATABASE WPI Section Ch, Week 200152 Derwent Publications Ltd., London, GB; Class A96, AN 2001-478264 XP002372935 & JP 2001 139796 A (PIGEON KK) 22. Mai 2001 (2001-05-22)

## Beschreibung

Verbraucherwünsche und Rheologie kosmetischer Produkte sind eng miteinander verknüpft. So wird. z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Sonnenschutzfilter) und auch die Lagerstabilität der Formulierung steht in enger Abhängigkeit zu den rheologischen Eigenschaften des Produktes. Im kosmetischen Bereich kommt daher den Verdickungsmitteln und Gelbildnern eine tragende Rolle zu.
Eine Reihe von Patentschriften beschreiben den Einsatz von Polyetherestern als Verdickungsmittel. Stand der Technik ist die Verwendung von Fettsäureestern von langkettigen Polyethern, beispielsweise von Polyethylenglykol 6000 Distearat. In US-4,774,017 werden Polyethylen-glycol-polypropylen-glycol Monoether als konsistenzgebende Komponente, in DE 37 26 015 die Umsetzungsprodukte von Polyalkoholen mit Fettsäuren, beispielsweise Pentaerythritolfettsäureester und deren verdickende Wirkung, beschrieben. US 5,129,462 beschreibt Shampoo-Formulierungen, enthaltend Polyethylenglycol-Polyolfettsäureester, insbesondere PEG Pentaerythritolfettsäureester als Verdickungsmittel. Die Verarbeitung und Formulierbarkeit dieser Verbindungsklasse ist durch deren hohen Schmelzpunkte bzw. Stockpunkte beeinträchtigt. DE 101 24 547 und WO 01/08481 beschreiben die Verwendung von alkoxylierten Polyglycerinestern als Emulgatoren in der Emulsionspolymerisation bzw. die Verwendung in Pestizidzubereitungen.

Gegenstand der Erfindung sind kosmetische und pharmazeutische Zubereitungen enthaltend einen oxalkylierten Polyglycerinester der Formel (1) 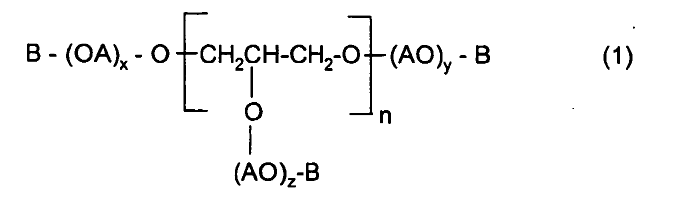 worin A eine Gruppe der Formel -C₂H₄-,
B Wasserstoff oder eine Gruppe der Formel -COR bedeuten,
wobei mindestens ein Symbol B eine Gruppe der Formel -COR ist, R C₇-C₂₁-Alkyl oder C₇-C₂₁-Hydroxyalkyl oder C₂-C₂₁-Alkenyl, n eine Zahl von 1,8 bis 5 und x, y und z Zahlen von 0 bis 100 bedeuten, wobei die Summe von x, y und z 130 bis 170 beträgt.
Alkoxylierte Polyglycerinester gemäß Formel (1) weisen gegenüber dem Stand der Technik, beispielsweise PEG 6000 Distearat, eine verbesserte Verdickerleistung auf und haben einen niedrigeren Stockpunkt, was anwendungstechnisch von großem Vorteil ist. Es sind überwiegend weiche Wachse, die mit weiteren Komponenten, beispielsweise Wasser, mehrwertigen Alkoholen, insbesondere Glykolen wie Propylen-, Butylen-, Hexylenglykol, Polyethylenglykolen, alkoxylierten Alkoholen mit C₁-C₂₂-Alkylresten und 1 bis 50 EO- oder PO-Einheiten, ethoxylierten Partialglyceriden, beispielsweise PEG-7-Glycerylcocoat und Glycerin fließbare, gut handhabbare Produkte ergeben.

Die Herstellung dieser oxalkylierten Polyglycerinester erfolgt entweder durch Oxalkylierung eines Polyglycerins und anschließende Veresterung oder durch Veresterung des Polyglycerins und anschließende Oxalkylierung.

Die erfindungsgemäß eingesetzten alkoxylierten Polyglycerinester werden in mehreren Reaktionsstufen hergestellt. Die Synthese der Polyglycerine bzw. Oligoglycerine, Diglycerin, Triglycerin, Tertraglycerin bis Decaglycerin erfolgt in bekannter Weise durch Polykondensation von Glycerin in Gegenwart von Katalysatoren, beispielsweise reduzierende Phosphorsäuren, Alkalihydroxide, Alkalicarbonate, Alkalibicarbonate, Alkalialkoholate und Alkoxide bei Temperaturen von 190°C bis 270°C. Unter Austrag von Kondensationswasser erfolgt innerhalb von 8 bis 72 Stunden die Bildung der Polyglycerine. Die Hydroxylzahl (OH-Zahl) eines solchen Reaktionsgemisches liegt beispielsweise bei 1072 mg KOH/g für ein Oligomerengemisch, das im Mittel einem Polyglycerin-4 entspricht. Die Zahl n als Maß für den Kondensationsgrad liegt zwischen 1.8 und 5.

Die Alkoxylierung, insbesondere die Ethoxylierung der Polyglycerine bzw. Oligoglycerine erfolgt bei 130 bis 190°C, bevorzugt 160°C in Gegenwart eines basischen Katalysators, beispielsweise NaOH, nach Trocknung bei 100°C und 20 mbar Vakuum (ca. 0,5 Stunden), wobei Alkoxid, vorzugsweise Ethylenoxid bei einem Druck von 1 bar bis 6 bar, insbesondere bei 4 bar bis 6 bar innerhalb von 15 Stunden zudosiert wird. Die erhaltenen ethoxylierten Polyglycerine bzw. Oligoglycerine haben einen Gesamt-EO-Grad (x+y+z) von 130 bis 170 bezogen auf die mittlere Molekularmasse des Polyglycerins.

Das hergestellte ethoxylierte Poly-/Oligoglycerin wird nach Abkühlen des Reaktionsgemisches auf 60 -100°C mit einem Katalysator, z.B. Alkylbenzolsulfonsäure versetzt und durch Säurezusatz, bevorzugt unterphosphoriger Säure oder Phosphorsäure der pH-Wert auf 4 - 5 (10 % wässrig) eingestellt. Anschließend wird durch Zusatz einer Fettsäure, beispielsweise Stearinsäure, Isostearinsäure, 12-Hydroxystearinsäure, Kokosfettsäure, Laurinsäure, Ölsäure oder deren Alkylester, Chloride oder Anhydriden bei einer Reaktionstemperatur von 160 bis 230°C und einer Reaktionsdauer von 10 bis 35 Stunden verestert. Der molare Anteil an Fettsäure bzw. Fettsäurederivaten kann beliebig gewählt werden, mindestens eine OH-Gruppe des oxalkylierten Polyglycerins muss aber verestert sein.

Die Herstellweise dieser alkoxylierten Poly-/Oligoglycerin-Fettsäureester kann auch so variiert werden, dass die Poly-/Oligoglycerine zuerst im entsprechenden Molverhältnis mit Fettsäure verestert und dann alkoxyliert werden.

Bedingt durch dieses Herstellverfahren handelt es sich bei den erfindungsgemäß benutzten Polyglycerinderivaten um Mischungen von Verbindungen der oben genannten Formel mit unterschiedlichem Wert für n, d.h. es kommen auch Gemische mit einem Gehalt an Monoglycerinester in Frage.

Diese Polyglycerinester eignen sich als Verdicker und Dispergiermittel für wässrige, wässrig-alkoholische und wässrig-tensidische Zubereitungen und als Emulgatoren, Suspendiermittel mit verdickender Wirkung und Konsistenzgeber für Emulsionen und Suspensionen.

Bei den Zubereitungen, Emulsionen und Suspensionen handelt es sich um kosmetische und pharmazeutische Mittel, wie z.B. Shampoos, Duschbäder, Duschgels, Schaumbäder, Gels, Lotions, Cremes und Salben.

Die erfindungsgemäßen Zubereitungen, Emulsionen und Suspensionen enthalten, bezogen auf die fertige Formulierung, bevorzugt 0,05 bis 10 Gew.-%, besonders bevorzugt 0,1 bis 5 Gew.-%, insbesondere bevorzugt 0,5 bis 3 Gew.-%, der beschriebenen alkoxylierten Polyglycerinester.

Die erfindungsgemäßen Mittel können als weitere Hilfs- und Zusatzstoffe alle üblichen anionischen, kationischen, zwitterionischen, nicht-ionischen und amphoteren Tenside, sowie weitere in der Kosmetik gebräuchliche Zusätze, wie z.B. Überfettungsmittel, Stabilisatoren, biogene Wirkstoffe, Glycerin, Konservierungsmittel, Perlglanzmittel, Farb- und Duftstoffe, Lösungsmittel, Trübungsmittel, weitere Verdickungsmittel und Dispergiermittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden

Die Gesamtmenge der in den erfindungsgemäßen Mitteln eingesetzten Tenside kann, bezogen auf das fertige Mittel, zwischen 5 bis 70 Gew.-%, bevorzugt zwischen 10 und 40 Gew.-%, besonders bevorzugt zwischen 12 und 35 Gew.-%, bezogen auf 100 % Wirksubstanz, betragen.
Als anionische waschaktive Substanzen seien genannt: (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside in den erfindungsgemäßen Mitteln liegt bevorzugt im Bereich von 7 bis 30 Gew.-%, besonders bevorzugt 10 bis 25 Gew.-%, insbesondere bevorzugt 12 bis 22 Gew.-%.

Geeignete kationische Tenside sind beispielsweise quartäre Ammonium-Salze wie Di-(C₁₀-C₂₄)-Alkyl-dimethylammoniumchlorid oder -bromid, vorzugsweise Di-(C₁₂-C₁₈)-Alkyl-dimethylammoniumchlorid oder -bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyl-dimethylbenzylammoniumchlorid oder -bromid, vorzugsweise (C₁₂-C₁₈)-Alkyl-dimethylbenzylammoniumchlorid; N-(C₁₀-C₁₈)-Alkyl-pyridiniumchlorid oder -bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyoylaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside in den erfindungsgemäßen Mitteln liegt bevorzugt im Bereich von 1 bis 10 Gew.-%, besonders bevorzugt 2 bis 7 Gew.-%, insbesondere bevorzugt 3 bis 5 Gew.-%.
Als nichtionische Tenside, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkylolamide, (Fettsäureamidopolyethylenglykole); N-Alkyl-, N-Alkoxypolyhydroxyfettsäureamid, Saccharoseester; Sorbitester und der Polyglykolether.

Der Gewichtsanteil der nichtionischen Tenside in den erfindungsgemäßen Mitteln liegt bevorzugt im Bereich von 1 bis 20 Gew.-%, besonders bevorzugt 2 bis 10 %, insbesondere bevorzugt 3 bis 7 Gew.-%.

Bevorzugte Amphotenside sind: N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol®, Steinapon®), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Der Gewichtsanteil der amphoteren Tenside in den erfindungsgemäßen Mitteln liegt bevorzugt im Bereich von 0,5 bis 20 Gew.-%, besonders bevorzugt 1 bis 10 Gew.-%.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind Laurylsulfat, Laurethsulfat, Cocoamidopropylbetain, Natriumcocoylglutamat, Di-natriumlaurethsulfosuccinat und Cocosfettsäurediethanolamid.
Die erfindungsgemäßen Zubereitungen können außerdem weitere in der Kosmetik gebräuchliche Zusätze wie Überfettungsmittel, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.
Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure.

Als Perlglanzmittel kommen beispielsweise Glycoldistearinsäureester, wie Ethylenglycoldistearat, aber auch Fettsäuremonoglykolester in Betracht.
Als Farbstoffe können die für kosmetische Zwecke geeigneten und zugelassenen Substanzen verwendet werden.
Als weitere Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol 934, -940, -941, -956, -980, -981, -1342, -1382, Ethylenglykolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat,
N,N-Dihydrocarbyl (C₁₂-C₂₂)- insbesondere (C₁₆-C₁₈)-amidobenzoesäure und deren lösliche Salze, N,N-di(C₁₆-C₁₈)-amidobenzoesäure und deren Derivate.
Die Dispergiermittel werden, bezogen auf das fertige Mittel, in Konzentrationen von bevorzugt 0,5 bis 10 Gew.-%, besonders bevorzugt von 0,5 bis 5 Gew.-%, insbesondere bevorzugt von 1 bis 4 Gew.-%, eingesetzt.
Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol.

Als Trägermaterialien in Betracht kommen pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und CelluloseDerivate.

Der nichtwässrige Anteil der Emulsionen, der sich weitgehend aus dem Emulgator, dem Verdicker und dem Ölkörper zusammensetzt, liegt üblicherweise bei 5 bis 95 %, vorzugsweise bei 15 bis 75 Gew.-%. Daraus folgt, dass die Emulsionen 5 bis 95 Gew.-% und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Ölkörper, Co-Emulgatoren, Überfettungsmittel, Fette, Wachse, Stabilisatoren, biogene Wirkstoffe, Glycerin, Konservierungsmittel, Farb- und Duftstoffe enthalten.

Als Ölkörper kommen beispielsweise Guerbetalkohole auf Basis von Fettalkoholen mit 6 bis 18, vorzugsweise 8 bis 10 Kohlenstoffatomen, Ester von linearen (C₆-C₁₃)-Fettsäuren mit linearen (C₆-C₂₀) Fettalkoholen, Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis (C₆-C₁₀)-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht. Der Anteil der Ölkörper am nichtwässrigen Anteil der Emulsionen kann 5 bis 95 und vorzugsweise 15 bis 75 Gew.-% ausmachen.
Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmonound -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, diund tri-Alkylquats und deren polymere Derivate.
Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.
Die Herstellung der Emulsionen kann in bekannter Weise, d.h. beispielsweise durch Heiß-, Heiß/Kalt- bzw. PIT-Emulgierung erfolgen.

### Beispiel 1: Herstellung von ethoxiliertem (150 EO) Diglycerin, verestert mit 3 Mol Stearinsäure

a) Herstellung von ethoxiliertem (150 EO) Diglycerin Das Rohprodukt (Diglycerin) wurde mit einem basischen Katalysator (NaOH) versetzt und anschließend.bei 90 - 100°C/20 mbar eine halbe Stunde lang getrocknet. Darauffolgend wurden bei ca. 160°C und einem Druck von 5 bar 150 Mol EO pro Mol Diglycerin innerhalb von 15 Stunden zudosiert. Am Ende der Nachreaktionszeit (ca. 2 Stunden) wurde das Produkt abgekühlt und entgast.
b) Herstellung von ethoxiliertem (150 EO) Polyglycerol-2 Tristearat 250,00 g ethoxiliertes (150 EO) Diglycerin wurden in einen Rührbehälter (mit N2-Durchleitung und Wasserauskreiser) gegeben und mit 1,26 g Alkylbenzolsulfonsäure (0,45 % vom Gesamtansatz) und 0,67 g unterphosphoriger Säure (0,24 % vom Gesamtansatz) versetzt. Anschließend wurde 30,40 g Stearinsäure (Molverhältnis ethoxyliertes Diglycerin :
   Stearinsäure = 1 : 3) zugegeben und die Reaktionsmischung unter Rühren auf 230°C erhitzt.
   Nach 30 Stunden Reaktionsdauer wurde eine OH-Zahl von 0,45 mg KOH/g erreicht.

Das Produkt ist ein weiches Wachs mit einem Schmelzpunkt von 38°C. Die übrigen in Tabelle 1 gezeigten Versuchsprodukte wurden ausgehend von Diglycerin oder Tetraglycerin durch Umsetzung mit EO und Stearinsäure bzw ölsäure in analoger Weise hergestellt.

Die herausragende Verdickungsleistung der alkoxylierten Polyglycerinester in einem wässrigen Tensidsystem wird in Tabelle 1 dem Verdickungsvermögen herkömmlicher Verdickungsmittel gegenübergestellt.

**Tabelle 1:**

| gemessene Viskositäten in mPa.s einer wässrigen Tensidlösung enthaltend 15 Gew.-% einer Mischung aus C₁₂/C₁₄-Alkyldiglykolethersulfat-Na und Cocoamidopropylbetain (Gewichtsverhältnis 8 zu 2) und Verdickungsmittel in der angegebenen Menge, eingestellt mit Zitronensäure/NaOH auf pH 6. | | | | | | | | |
|---|---|---|---|---|---|---|---|---|
| Verdicker | 1,5 Gew.-% | 2 Gew.-% | 2,5 Gew.-% | 3 Gew.-% | 3,5 Gew.-% | 4 Gew.-% | 4,5 Gew.-% | 5 Gew.-% |
| PEG 6000-Distearat - | - | - | 1110 | 4670 | 20000 | 29700 | 46000 | 81500 |
| PEG 120-methyl-glukose-di-oleat | 26 | 138 | 1650 | 8700 | 27200 | 12900 | - | |
| Diglycerin+150EO-tristearat | 7800 | 69000 | 163000 | >200000 | | | | |
| Polyglycerol-4+150 EO-pentastearat | | 670 | 9300 | 80000 | 144000 | >200000 | >200000 | >200000 |
| Diglycerin + 150 EO- distearat | - | 290 | 4300 | 24000 | 51000 | 111000 | 180000 | >200000 |

Nachfolgende Anwendungsbeispiele sollen den Gegenstand der Erfindung näher erläutern, ohne ihn darauf einzuschränken. Die Prozentangaben verstehen sich als Gewichtsprozent.

| Schampoo-/Duschbadrezepturen | | |
|---|---|---|
| a) | ®Genapol LRO fl., WS 28 % | 44,4 % |
| | (C₁₂/₁₄-Alkyldiglykolethersulfat, Na) | |
| | ®Genagen CAB 818, WS 30 % | 10,0 % |
| | (Cocoamidopropylbetain) | |
| | Wasser | ad 100 % |
| | Diglycerin + 150 EO distearat | 2,57 % |
| | Zitronensäure/NaOH | |

Die Formulierung weist eine Viskosität von 4300 mPas auf.

| | | |
|---|---|---|
| b) | Genapol LRO fl., WS 28 % | 44,4 % |
| | (C12/14-Alkyldiglykolethersulfat, Na) | |
| | Genagen CAB 818, WS 30 % | 10,0 % |
| | Wasser | ad 100 % |
| | Diglycerin + 150 EO tristearat | 1,5 % |
| | Zitronensäure/NaOH | |

Die Formulierung weist eine Viskosität von 3800 mPas auf.

| | | |
|---|---|---|
| c) | Genapol LRO fl., WS 28 % | 44,4 % |
| | (C12/14-Alkyldiglykolethersulfat, Na) | |
| | Genagen CAB 818, WS 30 % | 10,0 % |
| | (Cocoamidopropylbetain) | |
| | Wasser | ad 100 % |
| | Tetraglycerin + 150 EO distearat | 5,5 % |
| | Zitronensäure/NaOH | |

Die Formulierung weißt eine Viskosität von 4100 mPas auf.

| | | |
|---|---|---|
| d) | Genapol LRO fl., WS 28% | 44,4 % |
| | (C₁₂/₁₄-Alkyldiglykolethersulfat, Na) | |
| | Genagen CAB 818, WS 30 % | 10,0 % |
| | (Cocoamidopropylbetain) | |
| | Wasser | ad 100 % |
| | Tetraglycerin + 150 EO tristearat | 4,0 % |
| | Zitronensäure/NaOH | |

Die Formulierung weißt eine Viskosität von 2680 mPas auf.

| | | |
|---|---|---|
| e) | Genapol LRO fl., WS 28% | 44,4 % |
| | (C12/14-Alkyldiglykolethersulfat, Na) | |
| | Genagen CAB 818, WS 30% | 10,0 % |
| | (Cocoamidopropylbetain) | |
| | Wasser | ad 100 % |
| | Tetraglycerin + 150 EO tetrastearat | 2,5 % |
| | Zitronensäure/NaOH | |

Die Formulierung weißt eine Viskosität von 4200 mPas auf.

| | | |
|---|---|---|
| f) | Genapol LRO fl., WS 28% | 44,4 % |
| | (C₁₂/₁₄-Alkyldiglykolethersulfat, Na) | 10,0 % |
| | Genagen CAB 818, WS 30 % (Cocoamidopropylbetain) | |
| | Wasser | ad 100 % |
| | Tetraglycerin + 150 EO pentastearat Zitronensäure/NaOH | 2,5 % |

Die Formulierung weißt eine Viskosität von 4300 mPas auf.
Die Viskosität wurde bei 20°C mit einem Brookfield Viskosimeter Typ RVT bei 20 Upm gemessen.

## Patentansprüche

1. Kosmetische und pharmazeutische Zubereitungen enthaltend einen oxalkylierten Polyglycerinester der Formel (1) 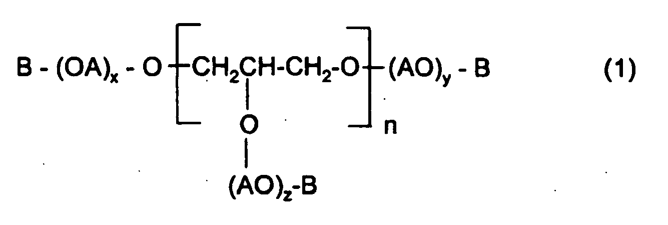 worin A eine Gruppe der Formel -C₂H₄-,
B Wasserstoff oder eine Gruppe der Formel -COR bedeuten,
wobei mindestens ein Symbol B eine Gruppe der Formel -COR ist, R C₇-C₂₁-Alkyl , C₇-C₂₁-Hydroxyalkyl oder C₂-C₂₁-Alkenyl, n eine Zahl von 1,8 bis 5 und x, y und z Zahlen von 0 bis 100 bedeuten, wobei die Summe von x, y und z 130 bis 170 beträgt.

2. Kosmetische und pharmazeutische Zubereitungen nach Anspruch 1, **dadurch gekennzeichnet, dass** sie 0,05 bis 10 Gew.-% des oxalkylierten Polyglycerinesters der Formel (1) enthalten.

3. Kosmetische und pharmazeutische Zubereitungen nach Anspruch 1 oder 2, **dadurch gekennzeichnet, dass** diese bei Raumtemperatur fließfähig sind.

4. Kosmetische und pharmazeutische Zubereitungen nach Anspruch 1, **dadurch gekennzeichnet, dass** es sich um Shampoos, Duschbäder, Duschgels, Schaumbäder, Gels, Lotions, Cremes oder Salben handelt.

## Claims

1. A cosmetic or pharmaceutical preparation comprising an oxalkylated polyglycerol ester of the formula (1) 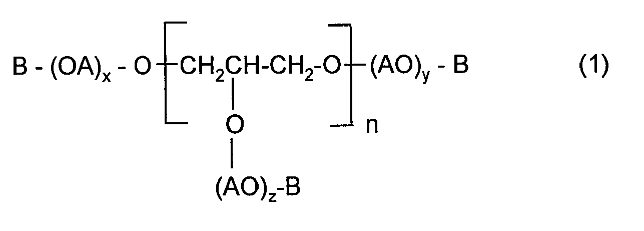 in which A is a group of the formula -C₂H₄-,
B is hydrogen or a group of the formula -COR,
where at least one symbol B is a group of the formula -COR, R is C₇-C₂₁-alkyl, C₇-C₂₁-hydroxyalkyl or C₂-C₂₁-alkenyl, n is a number from 1.8 to 5 and x, y and z are numbers from 0 to 100, where the sum of x, y and z is 130 to 170.

2. The cosmetic or pharmaceutical preparation as claimed in claim 1, which comprises 0.05 to 10% by weight of the oxalkylated polyglycerol ester of the formula (1).

3. The cosmetic or pharmaceutical preparation as claimed in claim 1 or 2, which is flowable at room temperature.

4. The cosmetic or pharmaceutical preparation as claimed in claim 1, which is in the form of a shampoo, shower preparation, shower gel, foam bath, gel, lotion, cream or ointment.

## Revendications

1. Préparations cosmétiques et pharmaceutiques, contenant un poly(ester de glycérol) oxalkylé de formule (1) 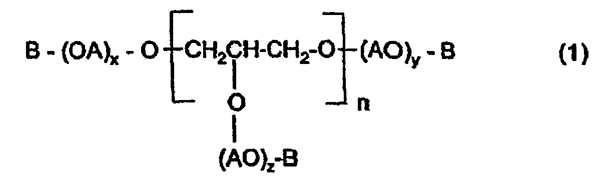 où A signifie un groupe de formule -C₂H₄-,
B signifie hydrogène ou un groupe de formule -COR,
où au moins un symbole B signifie un groupe de formule -COR, R signifie C₇-C₂₁-alkyle, C₇-C₂₁-hydroxyalkyle ou C₂-C₂₁-alcényle, n vaut un nombre de 1,8 à 5 et x, y et z valent des nombres de 0 à 100, la somme de x, y et z valant 130 à 170.

2. Préparations cosmétiques et pharmaceutiques selon la revendication 1, **caractérisées en ce qu'**elles contiennent 0,05 à 10% en poids du poly(ester de glycérol) oxalkylé de formule (1).

3. Préparations cosmétiques et pharmaceutiques selon la revendication 1 ou 2, **caractérisées en ce qu'**elles sont aptes à l'écoulement à température ambiante.

4. Préparations cosmétiques et pharmaceutiques selon la revendication 1, **caractérisées en ce qu'**il s'agit de shampooings, de bains douche, de gels douche, de bains mousse, de gels, de lotions, de crèmes ou de pommades.
